# EUROPEAN PATENT APPLICATION

(11) **EP 4 467 959 A1**
(43) Date of publication of application: **27.11.2024**
(21) Application number: 23175688.3
(22) Date of filing: 26.05.2023
(51) Int. Cl.: G01N 1/31, G01N 33/543, G01N 21/64, G01N 21/03, G01N 21/77, G01N 21/84

(54) **SAMPLE HOLDER FOR A BIOLOGICAL SAMPLE AND METHOD FOR ANALYSING THE BIOLOGICAL SAMPLE**

(71) Applicant: Leica Microsystems CMS GmbH, 35578 Wetzlar (DE)
(72) Inventor: Schlaudraff, Falk, 35578 Wetzlar (DE); Schlicker, Oliver, 35578 Wetzlar (DE)
(74) Representative: Schaumburg und Partner Patentanwälte mbB

(57) **Abstract**

A sample holder (100) for a biological sample (106) is provided, the sample (106) including at least one first target analyte, the sample holder (100) comprises a sample area (105) configured to hold the biological sample (106), and at least a first control area (102) comprising at least one first control analyte (200a-200d) identical to the one first target analyte. In another aspect, a method for analysing the biological sample (106) is provided.

## Description

### Technical field

The invention relates to a sample holder for a biological sample including at least a first target analyte and a method for analysing the biological sample provided on the sample holder.

### Background

In the art, systems are known for analysing biological samples such as tissue sections to determine the spatial distribution of target analytes within the biological samples. This generally relies on staining with optically detectable, in particular fluorescent, markers that are specific to the respective analytes. The biological samples are often provided on sample holders, such as microscope slides, in order to simplify handling of a large number of individual biological samples.

A particularly important aspect of this analysis relies on successfully staining these samples repeatedly. Thus, when analysing the biological samples by means of staining with markers, it is often desirable to be able to check if the staining procedure was successful or not.

### Summary

It is an object to provide a sample holder for a biological sample and a method for analysing the biological sample that enables efficiently verifying of staining procedures.

The aforementioned object is achieved by the subject-matter of the independent claims. Advantageous embodiments are defined in the dependent claims and the following description.

A sample holder for a biological sample is provided, the biological sample including at least one first target analyte, the sample holder comprises a sample area configured to hold the biological sample, and at least a first control area comprising at least one first control analyte identical to the one first target analyte. In particular, the sample holder may be used during staining of the biological sample and during generating an optical readout of the biological sample. The sample holder enables controlling or verifying if during staining of the biological sample provided on the sample holder, optically detectable markers have successfully bound to the control analyte and therefore bound to the target analyte in the biological sample. The optically detectable markers may be fluorescent markers comprising an affinity reagent specific to the first target analyte and the first control analyte and at least one fluorescent dye, for example.

The first control analyte may be an oligonucleotide or protein directly attached to the first control area, for example. Further, the first control analyte may also be within a cell or a tissue attached to the first control area.

The first control area is preferably arranged at a fixed and/or predetermined location of the sample holder. This enables efficient repetitive analysis of the sample holder, in particular of a plurality of sample holders.

Preferably, the at least first control area comprises the first control analyte at a predetermined density. This means, that the first control analyte is arranged in the first control area at a known quantity per area of the control area. Preferably, the distribution of the first control analyte in the first control area is homogeneous. This enables determining the emission intensity of specific markers bound to the first control analyte in relation to their quantity bound to the first control analyte, e.g. the emission intensity of fluorescent light being emitted by excited fluorescent molecules or fluorescent dyes of at least one specific marker. Alternatively or additionally, several first control areas may be provided, for example, each first control area may comprise the first control analyte at a particular predetermined density different to the densities of the remaining first control areas. This enables determining the emission intensity of specific markers bound to the first control analyte over a range of densities.

Preferably, the sample holder comprises an identifier. The identifier enables identification of a particular one of the sample holders in a plurality of sample holders. The identifier might comprise an RFID.

It is particularly preferred, that the identifier is optically detectable, e.g. in the form of a barcode or a QR-code. This enables reading the identifier when generating an optical readout of the sample holder.

Preferably, the sample area is spatially separate from at least the first control area. This enables generating separate readouts from the sample area and the first control area. Additionally, the sample area and/or the control area may also be separate from the identifier.

Preferably, the sample holder comprises a base on which the sample area and the first control area are arranged and wherein the base is adapted to enable observation of the biological sample by means of an optical readout device, for example, a microscope. This enables particularly efficient handling of the sample holder and observation of the biological sample.

Preferably, at least a part of the sample holder comprising the sample area is optically transparent. In particular, the base is a planar glass slide, or a microscope slide. This enables particularly efficient handling of the sample holder and enables readily producing the sample holder.

Preferably, the at least first control area comprises at least one second control analyte, wherein, in particular, the at least one second control analyte is different from the at least one first control analyte. In particular, the second control analyte is identical to a second target analyte in the biological sample. This enables verifying the staining for at least a second control analyte.

Preferably, the sample holder comprises at least one second control area comprising at least one second control analyte, wherein the first control area is spatially separated from the second control area. This enables verifying the staining for at least a second control analyte. In particular, one of the control areas only comprises a single species of the control analytes. This enables verifying the staining for each control analyte independently, in particular without crosstalk between control analytes of different species.

In a further aspect, a method is provided for analysing a biological sample including at least one first target analyte. The biological sample is provided in the sample area of the sample holder. In particular, the method is used for analysing the staining of the biological sample including at least one first target analyte. The method comprises the following steps: step a): adding a staining reagent, at least comprising a first optically detectable marker specific to the first target analyte, to the sample area and to at least the first control area of the sample holder. Further, the method comprises step b): generating an optical readout of at least the first control area by means of an optical readout device, such as a microscope. Moreover, the method comprises step c): determining for at least the first control area an intensity parameter of at least the first optically detectable marker in the optical readout.

By adding the staining reagent to the sample area and the control area, the first marker may bind to the first target analyte and the first control analyte, respectively. In particular, the first target analyte is identical to the first control analyte. The first marker may therefore comprise an affinity reagent specific to the first target analyte and the first control analyte as well as a fluorescent label. The intensity parameter may be based on the intensity of the fluorescent emission light of the first marker. This enables determining based on the intensity parameter, if the staining of the biological sample and the control area was successful at all. For example, if the first optically detectable marker may not be detected in the optical readout, the staining may be determined as unsuccessful. Therefore, the intensity parameter can have a binary format, e.g. the staining was successful or it was unsuccessful. In this case, it can be encoded or stored as 1 or 0. Furthermore, the intensity parameter can depend on the amount of detected fluorescent emission light and can comprise a value being in the range between 0 and a maximum intensity value.

Preferably, the method further comprises the step d): generating at least one optical readout of the biological sample in the sample area and analysing the biological sample based on the determined intensity parameter.

Preferably, the step c) or the step d) comprises rejecting the stained biological sample in case that the intensity parameter is below a predetermined threshold or has a value being 0.

Preferably, the at least first control area comprises the first control analyte at a predetermined density, and wherein step c) comprises adjusting at least one optical readout parameter of the optical readout device based on the intensity parameter.

Preferably in a step e) at least the first optically detectable marker is removed or quenched. This enables repeating the staining of the biological sample.

Preferably, the steps a) to e) are iteratively repeated. For example, different optically detectable markers may be used in each iteration. This enables detecting a plurality of target analytes in the biological sample. In one embodiment, individual control areas may be used for each round of staining.

In a further aspect, an analysing system for analysing a biological sample comprising at least one first target analyte, configured to perform the method above is provided.

Another aspect is a database storing intensity parameters from optical readouts of at least the first optically detectable marker from at least the first control area generated in the method and for storing information about the respective sample holder.

Preferably, the database is further adapted to store optical readouts of the biological sample of the respective sample holder.

Preferably, the database is supplementable by means of artificial intelligence or machine learning or deep learning, or wherein the data contained in the database is analysed by means of artificial intelligence or machine learning or deep learning.

### Short Description of the Figures

Hereinafter, specific embodiments are described referring to the drawings, wherein:
- Figure 1: is a schematic view of a sample holder with a control area,
- Figure 2: is a schematic view of the control area with various control analytes, and
- Figure 3: is a flow diagram for a method to analyse a biological sample.

### Detailed Description

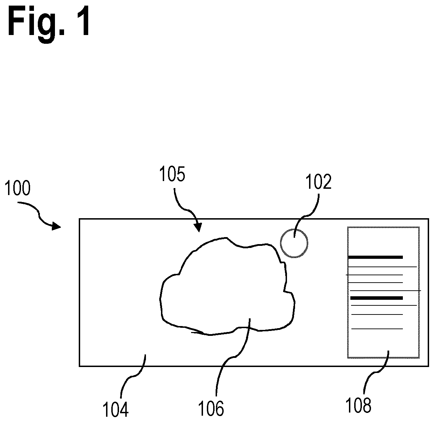

Figure 1 is a schematic view of a sample holder 100 with a control area 102. The sample holder comprises a base 104. Preferably, the base 104 is configured to enable observation of a biological sample 106, such as a tissue section, by means of an optical readout device. The optical readout device may be a microscope, a light microscope, a fluorescence light microscope, a widefield microscope, a scanning microscope, a confocal microscope, or an electron microscope, for example. The base 104 is preferably a microscope slide, for example, a planar glass slide. The control area 102 and the biological sample 106 within a sample area 105 are arranged on one side of the base 104. Thus, the control area 102 and/or the sample area 105 may be illuminated with illumination light from one side of the base 104 and observed by means of the microscope from the other side of the base 104, for example.

The control area 102 comprises at least one control analyte. The control analyte may be attached to the base 104 in the control area 102. The control analyte is, in particular, identical to a target analyte in the biological sample 106. This means, that the control analyte is of the same species as the target analyte, for example, the control analyte is the same protein as the target analyte. The sample holder 100 may comprise several control areas 104. In this case, each control area 104 may comprise a specific control analyte and/or each control area 104 may comprise the same control analyte but in different densities. Alternatively, each control area may comprise several different control analytes.

For example, the target analyte(s) of the biological sample 106 may be stained with optically detectable markers on the sample holder 100. In particular, at least one target analyte may be stained with a marker, the marker comprising an affinity reagent for specifically attaching the target analyte and a label for optically detecting the marker. The affinity reagent may be an antibody, for example and the label may be a fluorophore. Since the control analyte is the same as the target analyte in the biological sample, the marker binds to the target analyte as well as the control analyte. Thus, when staining the biological sample 106, the control area 102 of the sample holder 100 may be stained at the same time. This enables checking, if the staining procedure is successful or not, for example.

Preferably, the sample holder comprises an identifier 108, arranged on the base 104. The identifier 108 may be an optically detectable barcode, for example. Alternatively or in addition, the identifier 108 may be detectable by other means, for example, the identifier 108 may comprise a RFID-chip. The identifier 108 is particularly useful, when a plurality of different biological samples 106 are provided, each on one of the sample holders 100. By having a unique identifier 108 on each sample holder 100, the sample holders 100 and therefore the respective biological samples 106 can be identified individually.

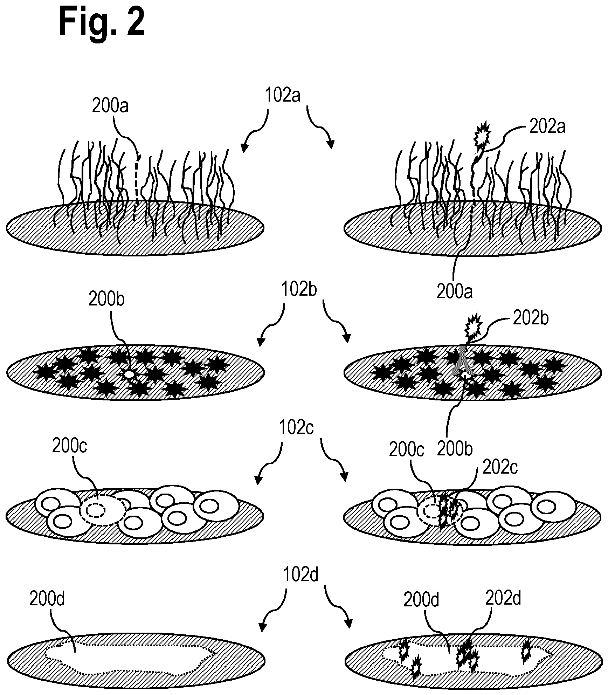

Figure 2 is a schematic view of embodiments of the control area 102 with different control analytes 200a, 200b, 200c, 200d. The control area 102a comprises oligonucleotides 200a as the control analyte. The oligonucleotide 200a may bind to a marker 202a comprising a complementary oligonucleotide as an affinity reagent.

The control area 102b comprises proteins 200b as the control analyte. The protein 200b may bind to markers 202b comprising an antibody specific to the protein 200b as an affinity reagent.

The control area 102c comprises cells 200c as the control analyte. The cell 200c may comprise an analyte, such as a protein, that markers 202c comprising an antibody specifically bind to.

The control area 102d comprises a tissue section 200d as the control analyte. The tissue section 200d may comprise an analyte, such as an oligonucleotide, that markers 202d comprising a complementary oligonucleotide bind to.

By applying markers 202a-202d for staining the biological sample 106, in particularly by binding specifically to the target analyte of the biological sample 106, to the sample area 105 and the control area 102 of the sample holder 100, the markers 202a-202d may bind to both, the target analyte and the respective control analyte 200a-200d. Thus, a successful staining may be determined by observing if the marker 202a-202d bound to the control analyte 200a-200d in the control area 102a-102d.

In particular in the case of the control analytes 200a, 200b the control analytes may be attached to the control area 102a, 102b in predetermined quantities.

In particular for the control analytes 200a, 200b in the control areas 102a, 102b, the control analytes 200a, 200b may be attached at predetermined densities, that means at predetermined and uniform amounts of control analyte 200a, 200b per unit area of the control area 102a, 102b.

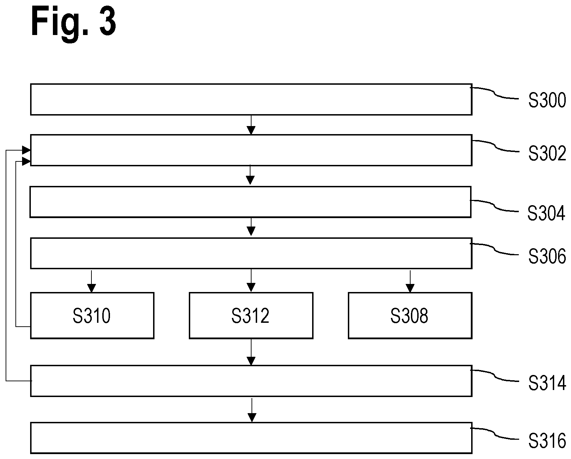

Figure 3 is a flow diagram for a method to analyse the biological sample 106, in particular, to analyse the staining of the biological sample 106. The biological sample 106 is provided in the sample area 105 of the sample holder 100. The method starts in step 5300. In step S302, a staining reagent is added to the sample area 105 and the control area 102. The staining reagent comprises optically detectable markers specific to a particular target analyte in the biological sample 106 and the respective control analyte 200a-200d in the control area 102a-102d. The biological sample 106 may comprise a plurality of target analytes, in which case the staining reagent may comprise plurality of markers 202a-202d that are each specific to a particular one of the target analytes. Similarly, the control area 102a-102d may comprise a plurality of respective control analytes 200a-200d. Alternatively, several control areas 102a-102d may be provided, each comprising a particular one of the control analytes 200a-200d. By applying the staining reagent, the markers 202a-202d bind to the respective target analytes and control analytes.

In a step S304, an optical readout of at least the control area 102 by means of an optical readout device. For example, the control area 102 may be imaged by means of a microscope.

In a step S306, an intensity parameter is determined for at least the first control area 102 in the optical readout of step S304. In case the optically detectable marker 202a-202d comprises fluorophores as labels, the intensity parameter may be the intensity of a fluorescent emission at an emission wavelength of the fluorophores, for example. The method ends in step S308, in case no intensity is detected and the intensity parameter is zero. In this case, the staining may be determined as unsuccessful.

Alternatively, the method may continue in a step S310, in case no intensity is detected and the intensity parameter is zero, in which case the method and the staining is repeated by starting with step S302.

Alternatively, the method may continue in step S312, if an intensity is detected, in particular, if the intensity parameter is above a predetermined threshold. Optionally, the step S312 may comprise adjusting the readout device based on the intensity parameter. For example, an excitation light intensity of the readout device, a spectral detection range of the readout device, or a detection duration or exposure time of the readout device may be adjusted. In a specific example, in case markers with green fluorescent protein (GFP) or red fluorescent protein (RFP) are used, the excitation light intensity may be adjusted for each, GFP and RFP, individually. Further, spectral unmixing parameters may be adjusted. Particularly, two makers with different labels that emit fluorescent light with overlapping wavelengths may be detected individually and separately by providing control areas that comprise control analytes to which either one marker binds. This enables determining the particular emission wavelengths for each marker individually.

In step S314, an optical readout of the sample area 105 with the biological sample 106 is generated. The biological sample 106, in particular the respective optical readout, may then be analysed based on the determined intensity parameter. For example, based on the intensity parameter, a quantitative analysis of the target analyte in the biological sample 106 may be carried out, in particular, when the respective control analyte 200a-200d is attached at a predetermined density. The method ends in step S316.

Alternatively, at least the biological sample 106 may be treated in order to remove or quench the markers bound to the biological sample 106 and the method may continue in step S302 with another staining cycle. This may continue iteratively, in order to stain the biological sample 106 with different markers in each staining cycle. Dedicated control areas 102 for each staining cycle and the respective control analytes 200a-200d may be provided on the sample holder 100.

An additional example of analysing the biological sample 106 based on the intensity parameter in step S314 relies on determining the intensity parameter of the markers without crosstalk. This may be achieved by providing a plurality of control areas 102, each with a single control analyte 200a-200d, such that when determining the intensity parameter in the readout of the control area 102 in step S306, the intensity parameter is determined without crosstalk to further markers. The intensity parameter determined in such a fashion may be used for unmixing of emission light from the markers in the optical readout generated in step S314. This is particularly useful in case several markers are used to detect a corresponding several target analytes in the biological sample 106.

In a further alternative, the staining reagent comprising the markers 202a-202d may be applied to the control area 102 together with binding agents unspecific to the respective control analyte, in order to determine the intensity parameter with unspecific binding partners present.

The method described above may be carried out by an analysing system configured to carry out the method. The system may comprise at least an optical readout device such as a microscope configured to receive the sample holder 100 and for generating an optical readout of the sample area 105 and the control area 102, and a liquid handling unit for applying at least the staining reagent to the sample area 105 and the control area 102. Optionally, the analysing system may further comprise a robotic handling unit for handling a plurality of sample holders 100 and shuttling the sample holders 100 between the optical readout device and the liquid handling unit.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100: Sample holder
- 102, 102a, 102b, 102c, 102d: Control area
- 104: Base of sample holder
- 105: Sample area
- 106: Biological sample
- 108: Identifier
- 200a, 200b, 200c, 200d: Control analyte
- 202a, 202b, 202c, 202d: Marker

## Claims

1. A sample holder (100) for a biological sample (106) including at least one first target analyte, the sample holder (100) comprising:
a sample area (105) configured to hold the biological sample (106), and
at least a first control area (102) comprising at least one first control analyte (200a-200d) identical to the one first target analyte.

2. The sample holder according to claim 1, wherein the at least first control area (102) comprises the first control analyte (200a-200d) at a predetermined density.

3. The sample holder according to one of the preceding claims, comprising an identifier (108).

4. The sample holder according to claim 3, wherein the identifier (108) is optically detectable.

5. The sample holder according to one of the preceding claims, wherein the sample area (105) is spatially separate from at least the first control area (102).

6. The sample holder according to one of the preceding claims, comprising a base (104) on which the sample area (105) and the first control area (102) are arranged and wherein the base (104) is adapted to enable observation of the biological sample (106) by means of an optical readout device.

7. The sample holder according to one of the preceding claims, wherein at least a part of the sample holder (100) comprising the sample area (105) is optically transparent.

8. The sample holder according to one of the preceding claims, wherein the at least first control area (102) comprises at least one second control analyte.

9. The sample holder according to one of the preceding claims, comprising a second control area (102) comprising at least one second control analyte, wherein the first control area is spatially separated from the second control area.

10. A method for analysing a biological sample (106) including at least one first target analyte, the biological sample (106) is provided in the sample area (105) of a sample holder (100) according to one of the preceding claims, the method comprising the following steps:
a) adding a staining reagent, at least comprising a first optically detectable marker (202a-202d) specific to the first target analyte, to the sample area and to at least the first control area (102) of the sample holder (100),
b) generating an optical readout of at least the first control area (102) by means of an optical readout device, and
c) determining for at least the first control area (102) an intensity parameter of at least the first optically detectable marker (202a-202d) in the optical readout.

11. The method according to claim 10 further comprising the step d): generating at least one optical readout of the biological sample (106) in the sample area (105) and analysing the biological sample (106) based on the determined intensity parameter.

12. The method according to claim 10 or 11, wherein the step c) or the step d) comprises rejecting the stained biological sample (106) in case that the intensity parameter is below a predetermined threshold.

13. The method according to one of the claims 10 or 11, wherein the at least first control area (102) comprises the first control analyte (200a-200d) at a predetermined density, and wherein step c) comprises adjusting at least one optical readout parameter of the optical readout device based on the intensity parameter.

14. The method according to one of the claims 10 to 13, wherein in a step e) at least the first optically detectable marker (202a-202d) is removed or quenched.

15. The method according to claim 14, wherein the steps a) to e) are iteratively repeated.

16. An analysing system for analysing a biological sample comprising at least one first target analyte, configured to perform the method according to one of the claims 10 to 15.

17. A database storing intensity parameters from optical readouts of at least the first optically detectable marker from the at least the first control area generated in the method according to one of the claims 10 to 15 and storing information about the respective sample holder.

18. The database according to claim 17, further adapted to store optical readouts of the biological sample of the sample holder.

19. The database according to claim 17 or 18, being supplementable by means of artificial intelligence or machine learning or deep learning, or wherein the data contained in the database is analysed by means of artificial intelligence or machine learning or deep learning.
